# EUROPEAN PATENT APPLICATION

(11) **EP 1 974 756 A1**
(43) Date of publication of application: **01.10.2008**
(21) Application number: 07707183.5
(22) Date of filing: 22.01.2007
(51) Int. Cl.: A61L 31/00, A61F 2/84

(54) **STENT**

(30) Priority: 23.01.2006 JP 2006014365
(71) Applicant: TERUMO KABUSHIKI KAISHA, Tokyo 151-0072 (JP)
(72) Inventor: NAGURA, Hiroaki, c/o TERUMO KABUSHIKI KAISHA, Kanagawa 259-0151 (JP); NAKAGAWA, Yuji, c/o TERUMO KABUSHIKI KAISHA, Kanagawa 259-0151 (JP)
(74) Representative: Dossmann, Gérard
(86) International application number: PCT/JP2007/050920
(87) International publication number: WO 2007/083797

(57) **Abstract**

It is intended to provide a stent having little or no risk of the breakage of a coating layer that is formed on the stent surface in the course of producing or transporting the stent or during the step of expanding the stent in clinical use. In other words, a stent to be implanted in a lumen in the living body wherein a layer including a physiologically active substance, a biodegradable polymer and a citric acid ester employed as a plasticizer is formed at least a part of the surface of the stent body.

## Description

### Technical Field

The present invention relates to a stent to be implanted at a stenosed or occluded portion generated in a lumen in a living body so as to maintain the opening state of the lumen.

### Background Art

In recent years, medical devices called stents have been used for the purpose of improving a stenosed or occluded portion generated in a lumen in a living body, such as blood vessel, bile duct, trachea, esophagus and urethra. A stent, used in order to treat various diseases generated by stenosis or occlusion of a blood vessel or other lumen, is a hollow tubular medical device which can be implanted in the lesion portion such as the stenosed portion and the occluded portion so as to dilate the lesion portion and to maintain the opening state of the lumen. For example, in relation to the coronary artery of a heart, a stent is used for the purpose of preventing restenosis after percutaneous transluminal coronary angioplasty (PTCA).

By implanting the hollow tubular medical device called stent in a blood vessel after a surgical operation, it has been succeeded to lower the rates of acute blood vessel occlusion and restenosis. Even where the stent is used, however, it is recognized by, for example, follow-up observation conducted half a year after implanted, that the portions where the stents are implanted are restenosed at a mean rate of around 20%. Thus, the problem of restenosis is still left as a serious problem to be solved.

To obviate this problem, recently, there have been proposed many trials for lowering the restenosis rate by loading a physiologically active substance such as a carcinostatic agent on the stent so that the physiologically active substance is sustainedly released locally in the portion of the lumen where the stent is implanting.

For example, as described in WO 2002/047731, a coating layer having a carcinostatic agent contained in a polyolefin elastomer is loaded on a stent, whereby local and sustained release of the physiologically active substance is realized.

On the other hand, in WO 2002/026281, acrylic polymers are used as the drug-containing polymers, whereby sustained release of an immunosuppressant is achieved. These physiologically active substance-containing polymers are stable in vivo, and are highly resistant to deterioration such as cracking, even after implanted in blood vessels. The drug-containing polymers, however, are non-biodegradable polymers, so that the polymers remain in the blood vessels even after dissipation of the physiologically active substance. Since these polymers are inflammation-irritant considerably, there is a fear that the polymers may cause restenosis or thrombotic complication in a late phase (Coronary Intervention, p. 30, Vol. 3, No. 5, 2004).

In order to dispel the fear, many researches are found to be made in which biodegradable polymers are used as the physiologically active substance-containing polymer. For example, in US-A-5464650, a drug such as dexamethasone and a biodegradable polymer such as polylactic acid are dissolved in a solvent, and the resulting solution is sprayed onto a stent, followed by evaporating off the solvent, to produce a stent from which the drug can be released sustainedly.

The biodegradable polymer disappears by being decomposed in and absorbed into the living body, at the same time as the release of the physiologically active substance or after the release of the physiologically active substance. Therefore, at the late phase, the risk of restenosis or thrombotic complication in the case of the biodegradable polymers is lower in comparison to the cases of the non-biodegradable polymers. In addition, among the biodegradable polymers, the aliphatic polyesters such as polylactic acid are high in bio-compatibility and are therefore widely researched as polymers for loading a physiologically active substance thereon.

However, the aliphatic polyesters such as polylactic acid are hard and brittle, and are poor in adhesion to stent bodies. Therefore, there is a risk of cracking or exfoliation of the coating layer on the stent in the course of producing or transporting the stent or during the step of expanding the stent in clinical use. Besides, when the coating layer is broken, there is a fear that the sustained release behavior desired of the physiologically active substance contained in the coating layer cannot be obtained. These problems are seen also in the cases of self-expandable stents.

Furthermore, where an aliphatic polyester such as polylactic acid is used as the drug-containing polymer, the aliphatic polyester such as polylactic acid is hard and brittle and is poor in adhesion to the stent body, which makes it impossible to satisfactorily carry out the operation of calking the stent to a balloon (the operation of attaching the stent to a balloon without using an adhesive or the like). Besides, where the operation of calking the stent to the balloon is not sufficiently conducted, in view of obviating the breakage of the coating layer on the surface of the stent, the grasping force for holding the stent on the balloon may be lowered and, further, the stent might be slipped-off.

### Disclosure of Invention

It is an object of the present invention to provide a stent in which there is no or little risk of the breakage of a coating layer formed on the surface of the stent in the course of producing or transporting the stent or during the step of expanding the stent in clinical use, while using a highly safe material for the stent.

More specifically, it is an object of the present invention to provide means as defined in the following (1) to (10).

(1) A stent to be implanted in a lumen in a living body, wherein a layer including a composition containing a physiologically active substance, a biodegradable polymer and a citric acid ester employed as a plasticizer is formed at least at a part of the surface of a stent body.

(2) The stent according to (1) above, wherein no base coat layer is present between the stent body and the layer which includes the composition and formed at least at a part of the surface of the stent body.

(3) The stent according to (1) or (2) above, wherein the citric acid ester employed as the plasticizer is at least one compound selected from the group including triethyl citrate, tributyl citrate, acetyltriethyl citrate, acetyltributyl citrate, trihexyl citrate, acetyltrihexyl citrate, butyryltrihexyl citrate, and analogs thereof.

(4) The stent according to any one of (1) to (3) above, wherein the citric acid ester employed as the plasticizer is contained in an amount in the range of 10 to 40 parts by mass based on 100 parts by mass of the biodegradable polymer.

(5) The stent according to any one of (1) to (4) above, wherein the biodegradable polymer is at least one polymer selected from the group including aliphatic polyesters, polyesters, polyacid anhydrides, polyorthoesters, polycarbonates, polyphosphazenes, polyphosphoric acid esters, polyvinyl alcohol, polypeptides, polysaccharides, proteins, and cellulose, a copolymer in which monomers constituting the polymers are copolymerized as desired, or a mixture of the polymer(s) and/or the copolymer(s).

(6) The stent according to any one of (1) to (5) above, wherein the biodegradable polymer is an aliphatic polyester.

(7) The stent according to (5) or (6) above, wherein the aliphatic polyester is at least one selected from the group including polylactic acid (PLA), polyglycolic acid (PGA), and lactic acid-glycolic acid copolymer (PLGA).

(8) The stent according to any one of (1) to (7) above, wherein the physiologically active substance is at least one compound selected from the group including carcinostatic agents, immunosuppressants, antibiotics, antirheumatics, antithrombotic agents, HMG-CoA reductase inhibitors, ACE inhibitors, calcium antagonists, antihyperlipidemic agents, integrin inhibitors, antiallergic agents, antioxidants, GPIIbIIIa antagonists, retinoids, flavonoids, carotenoids, lipid improving drugs, DNA synthesis inhibitors, tyrosine kinase inhibitors, antiplatelet agents, anti-inflammatory agents, bio-derived materials, interferons, and NO production promoting substances.

(9) The stent according to any one of (1) to (8) above, wherein the physiologically active substance is sirolimus or a sirolimus derivative.

(10) The stent according to any one of (1) to (9) above, wherein the stent body is made of a metal or a polymer.

The other objects, features and characteristics of the present invention will become apparent from a study of the preferred embodiments thereof illustrated by the following description and the accompanying drawings.

### Brief Description of Drawings

[FIG. 1]
   FIG. 1 is an example of side view of one embodiment of the stent according to the present invention.
[FIG. 2] FIG. 2 is an example of enlarged cross-sectional view taken along line A-A of FIG. 1.
[FIG. 3] FIG. 3 is an example of enlarged cross-sectional view taken along line A-A of FIG. 1.
[FIG. 4]

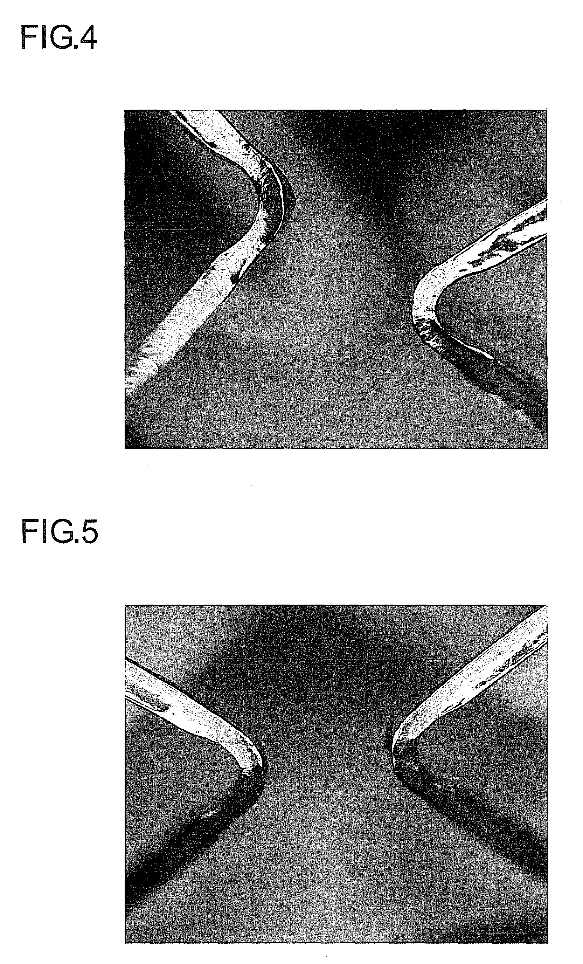

   FIG. 4 is an enlarged photograph of a surface of a stent after expansion in Example 1.
[FIG. 5]
   FIG. 5 is an enlarged photograph of a surface of a stent after expansion in Example 2.
[FIG. 6]

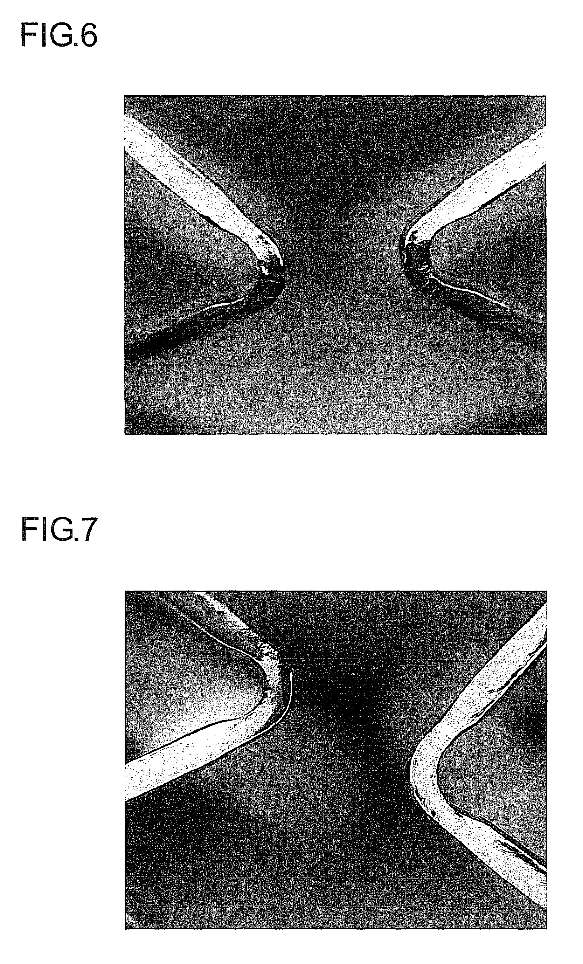

   FIG. 6 is an enlarged photograph of a surface of a stent after expansion in Example 3.
[FIG. 7]
   FIG. 7 is an enlarged photograph of a surface of a stent after expansion in Example 4.
[FIG. 8]

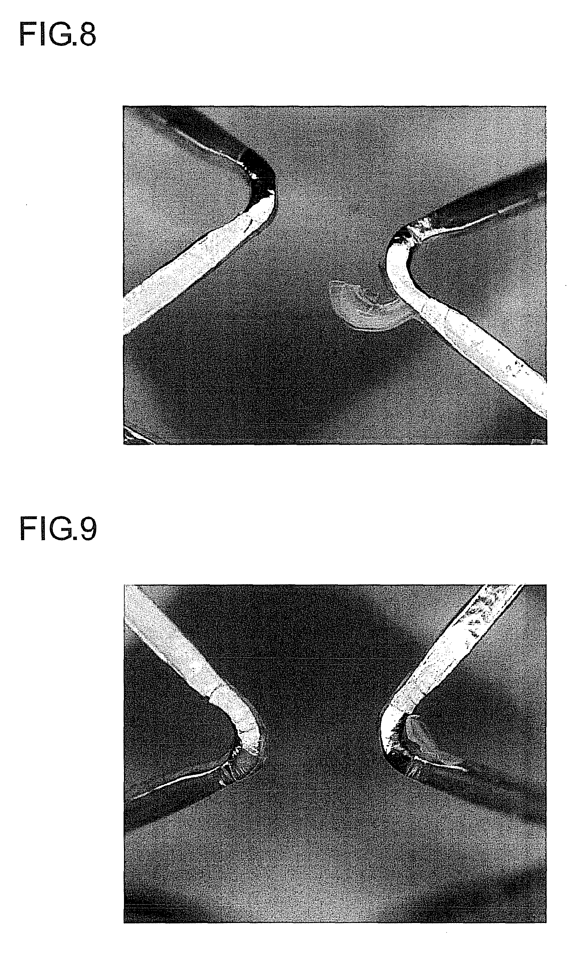

   FIG. 8 is an enlarged photograph of a surface of a stent after expansion in Comparative Example 1.
[FIG. 9]
   FIG. 9 is an enlarged photograph of a surface of a stent after expansion in Comparative Example 2.
[FIG. 10]

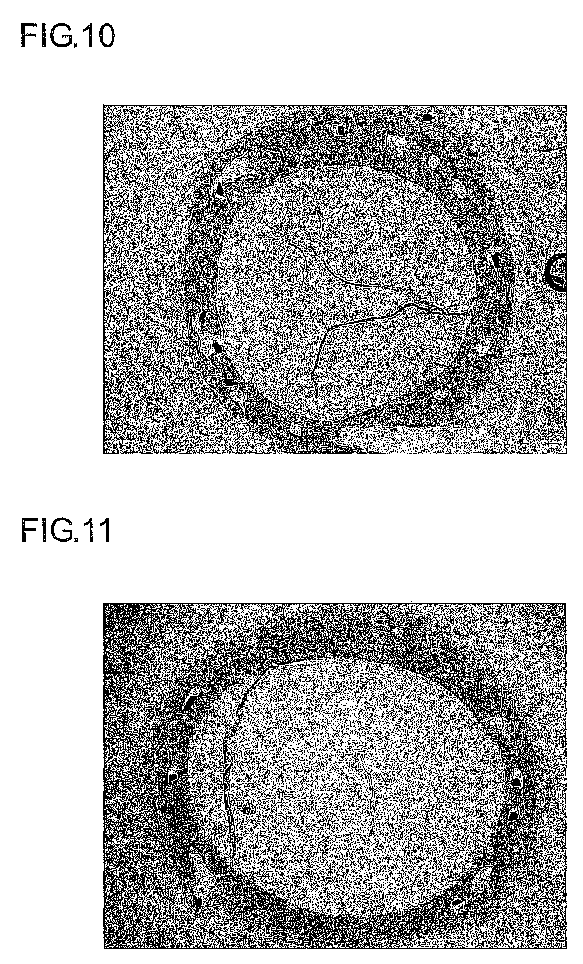

   FIG. 10 is a pathologic photograph of a stent taken one month after the implantation of a PLA/ATBC sample in Example 5.
[FIG. 11]
   FIG. 11 is a pathologic photograph of a stent taken one month after the implantation of a PLGA/ATBC sample in Example 5.
[FIG. 12]

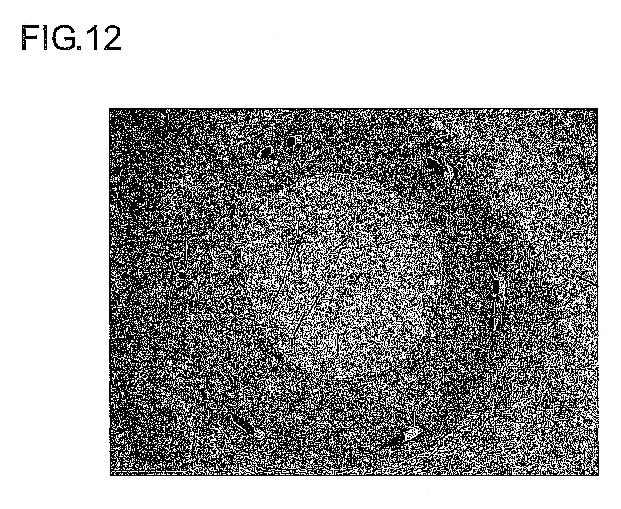

   FIG. 12 is a pathologic photograph of a stent taken one month after the implantation in Comparative Example 3 (a metal stent alone).

Incidentally, in the figures, symbol 1 denotes a stent, 11 denotes a roughly rhombic element, 12 denotes an annular unit, 13 denotes a link member, 2 denotes a filamentous member, 21 denotes an outside surface, 22 denotes an inside surface, 23 denotes a stent body, and 3 denotes a layer including a composition containing a physiologically active substance and a biodegradable polymer and a plasticizer.

### Best Mode for Carrying Out the Invention

Now, some embodiments of the present invention will be described in detail below.

The stent according to the present invention will be described in detail below, based on preferred embodiments shown in the accompanying drawings.

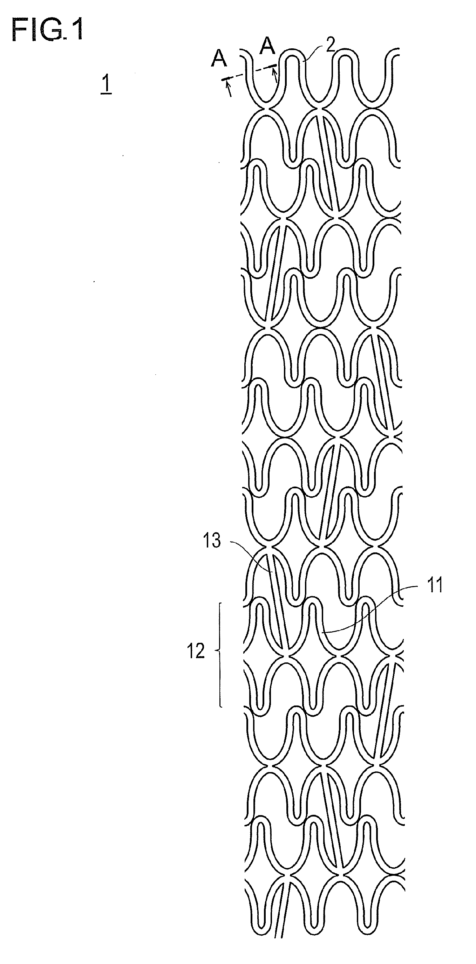

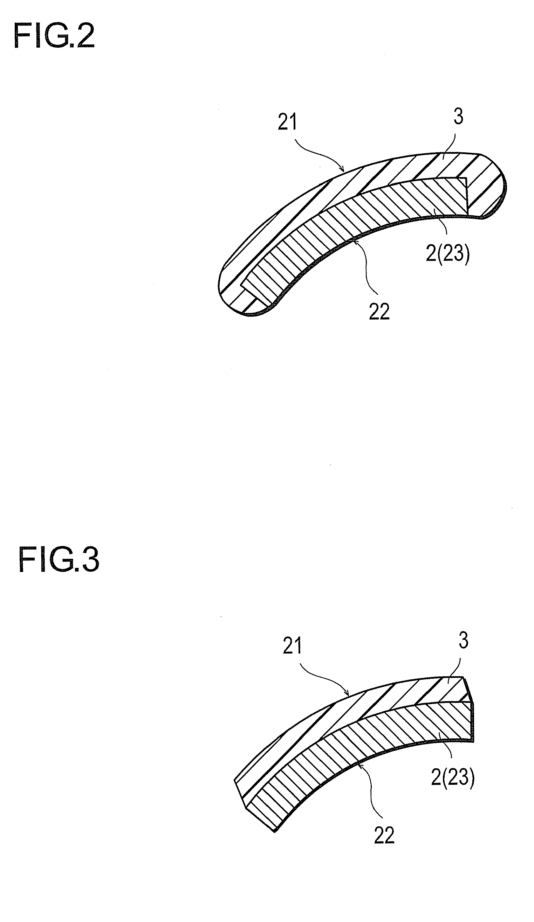

FIG. 1 is a side view showing one embodiment of the stent according to the present invention, and FIGS. 2 and 3 are enlarged cross-sectional views taken along line A-A of FIG. 1.

As shown in FIG. 2, in the stent 1 according to the present invention, a layer 3 including a composition containing a physiologically active substance, a biodegradable polymer and a citric acid ester employed as a plasticizer is formed on the surface of a filamentous member 2 constituting a stent body 23, in such a manner as to cover at least a part of the filamentous member 2.

As for the mode in which the layer 3 including the composition containing a physiologically active substance, a biodegradable polymer and a citric acid ester employed as a plasticizer is formed on the surface of the filamentous member 2 constituting the stent body 23, the layer 3 may be formed so as to cover the entire body of the filamentous member 2 or to cover a part of the filamentous member 2, or the layer 3 may be formed only on the upper surface of the outside surface 21 of the filamentous member 2 as shown in FIG. 3.

Further, the layer 3 is preferably formed at least at the portions of the filamentous member 2 which come into direct contact with the living body tissue. This ensures that the physiologically active substance released from the layer can be absorbed directly in the living body tissue, without flowing in a body fluid, for example, blood. Therefore, local dosing with the physiologically active substance can be made, and a more effective pharmacological activity can be attained.

Incidentally, FIGS. 2 and 3 each show an example of the section of the stent in the case where the stent body 23 is made of a metal, but the present invention is not limited to the case where the stent body 23 is made of a metal.

In the layer 3, the relative proportions (mass ratio) of the physiologically active substance and the biodegradable polymer is preferably in the range of 1:99 to 99:1, more preferably 20:80 to 80:20. It is thereby intended to load as large an amount as possible of the physiologically active substance, while taking into account the physical properties and the biodegradability of the layer containing the biodegradable polymer.

Besides, the additive amount of the citric acid ester is preferably in the range of 10 to 40 parts by mass, more preferably in the range of 10 to 30 parts by mass, based on 100 parts by mass of the biodegradable polymer. Where the additive amount of the citric acid ester is in the range of 10 to 40 parts by mass, the compatibility of the ester with the biodegradable polymer is good, an improvement in the physical properties of the layer containing the biodegradable polymer can be achieved suitably, and good flexibility can be imparted to the layer; consequently, it is possible to restrain or prevent the exfoliation of the layer at the time when or after the stent is implanted. This, naturally, is preferable.

Though depending on the shape and size of the stent, the thickness of the layer 3 is ordinarily determined such a range as not to markedly spoil the performances of the stent body, such as the performance of arriving at the lesion portion (delivery performance) and irritation to the blood vessel walls, and in such a range that the effect of release of the physiologically active substance is exhibited sufficiently. The mean thickness of the layer 3 is preferably in the range of 1 to 75 µm, more preferably 1 to 30 µm, and further preferably 1 to 10 µm. Where the mean thickness of the layer 3 is 1 to 75 µm, the effect of sustained release of the physiologically active substance when the stent is implanted in a lumen in a living body is excellent. In addition, the stent 1 itself is prevented from becoming too large in outer diameter, there is no risk of hampering the arrival of the stent at the lesion portion, the stent is not irritant to the blood vessel walls, and restenosis can be restrained or prevented.

In general, where a polymer is admixed with a plasticizer, the plasticizer enters into the molecules of the line-formed polymer, to facilitate mutual actions of the molecules. The plasticizer shows the function of so-called "rollers", like balls in a ball bearing.

More specifically, when a plasticizer having an appropriate degree of dipoles enters into the inside of the mutually tangled polymer chains, the intermolecular linkage (electromagnetic coupling, van der Waals force, etc.) that would hinder the mutual motions of the polymer chains is weakened, and the intermolecular distance in the mutually tangled polymer chains is enlarged. As a result of this, it is considered, the flexibility and compatibility are enhanced.

In the stent coated with the layer including the composition containing the citric acid ester employed as a plasticizer, the physiologically active substance and the biodegradable polymer as above-mentioned, the layer 3 shows flexibility owing to the above-mentioned plasticizing effect of the citric acid ester aimed at by the present invention, and, further, restraining or prevention of the breakage of the layer 3 at the time of expanding the stent can be expected. Furthermore, the calking operation at the time of attaching the stent to a balloon can be carried out satisfactorily, and such accidents as exfoliation of the layer 3 and slipping-off of the stent during a surgical operation can be prevented from occurring. Specifically, the stent according to the present invention has a characteristic feature in that the layer containing the physiologically active substance, the biodegradable polymer and the citric acid ester employed as a plasticizer is provided on the surface of the stent body. The use of the biodegradable polymer in the layer ensures that as the biodegradable polymer is decomposed in the living body, the physiologically active substance is gradually released into the living body, whereby an appropriate therapy can be achieved. In addition, since the plasticizing effect of the citric acid ester employed as a plasticizer imparts flexibility to the layer, it is possible to significantly restrain or prevent the breakage of the layer in the course of producing or transporting the stent or during the step of expanding the sent in clinical use. Therefore, according to the stent of the present invention, it is possible to prevent the layer from being exfoliated at the time of or after the operation of implanting the stent in a living body, and the layer can realize the desired sustained release of the physiologically active substance. Furthermore, the stent can be securely fixed to the balloon.

The citric acid ester in the present invention is preferably at least one plasticizer selected from the group including triethyl citrate, tributyl citrate, acetyltriethyl citrate, acetyltributyl citrate, trihexyl citrate, acetyltrihexyl citrate, butyryltrihexyl citrate and analogs thereof. Among these citric acid esters, particularly preferred are acetyltributyl citrate and butyryltrihexyl citrate which remarkably enhance the elongation (breaking elongation in tension) of the biodegradable polymer constituting the layer 3. The citric acid esters may be used either singly or in combination of two or more of them. These citric acid esters are low in reactivity for reaction with living body tissues, and can control the physical properties of the layer that contains the biodegradable polymer. Therefore, the layer shows flexibility, and it is possible to prevent such accidents as exfoliation of the layer and slipping-off of the stent during a surgical operation. Furthermore, it is considered that the citric acid esters have an appropriate degree of polarity and, hence, can sufficiently exhibit a plasticizing effect.

In addition, among the citric acid esters as above-mentioned, triethyl citrate, tributyl citrate, acetyltriethyl citrate, acetyltributyl citrate, and butyryltrihexyl citrate are substances that are approved as drug additives, food additives or medical-device additives and, hence, can be said that bio-compatibility is very high.

Therefore, triethyl citrate, tributyl citrate, acetyltriethyl citrate, acetyltributyl citrate, and butyryltrihexyl citrate are substances which are known to be particularly excellent in terms of cytotoxicity, mutagenicity and carcinogenicity regarded as properties required of a material to be implanted in the living body and of which the safety is guaranteed.

Now, the other component elements constituting the stent 1 will be described in detail below.

The stent body is a hollow cylindrical body opened at both ends thereof and extending in the longitudinal direction between both terminal end parts thereof. The side surface of the cylindrical body has a large number of cutouts linking the outside surface and the inside surface thereof, and the cylindrical body can be contracted and expanded in the radial direction through deformations of the cutouts. When the stent is implanted in a vessel such as a blood vessel or a living body lumen such as a bile duct, the cylindrical body maintains the shape thereof.

In the embodiment shown in FIG. 1., the stent body has basic units each having a roughly rhombic element 11 which is formed of an elastic filamentous material and is provided therein with a cutout. A plurality of the roughly rhombic elements 11 are disposed and interlinked continuously in the minor axis direction of the roughly rhombic shape, to constitute an annular unit 12. Each of the annular unit 12 is connected to the adjacent annular units through a filamentous elastic member 13. This structure ensures that the plurality of annular units 12 are disposed continuously in the axial direction thereof, in the state of being partly connected to each other. The stent body, with such a configuration, forms the hollow cylindrical body opened at both ends thereof and extending in the longitudinal direction between both terminal end parts thereof. The side surface of the cylindrical body is provided therein with the roughly rhombic cutouts so that the cylindrical body can be contracted and expanded in the radial direction thereof through deformations of the cutouts.

It is to be noted here, however, that the structure of the stent according to the present invention is not limited to the one embodiment in the figures. The present invention pertains to a concept widely including the structures of hollow cylindrical bodies each of which is opened at both ends thereof and extending in the longitudinal direction thereof between both terminal end parts thereof, has a side surface provided therein with a large number of cutouts linking the outside surface and the inside surface thereof, and can be contracted and expanded in the radial direction thereof through deformations of the cutouts. Thus, coil shapes are also included in the concept according to the present invention. Also, the sectional shape of the elastic filamentous material constituting the stent body is not limited to the rectangular shape as shown in FIGS. 2 and 3, and may be any of other shapes such as a circle, ellipses, and other polygons than rectangles.

Examples of the material of the stent body in the present invention include polymer materials, metallic materials, carbon fiber, and ceramics. These materials may be used either singly or in appropriate combinations, and are not particularly limited insofar as they have certain degrees of rigidity and elasticity. Among these materials which can be used, preferred are biro-compatible materials, more preferred are metallic materials, polymer materials, and carbon fiber, and further preferred are metallic materials and polymer materials.

Specific preferable examples of the polymer materials include polyolefins such as polyethylene and polypropylene, aromatic polyesters such as polyethylene terephthalate, aliphatic polyesters such as polylactic acid and polyglycolic acid, cellulose polymers such as cellulose acetate and cellulose nitrate, and fluorine-containing polymers such as polytetrafluoroethylene and tetrafluoroethylene-ethylene copolymer.

Preferable examples of the metallic materials include stainless steels, tantalum, tantalum alloys, titanium, titanium alloys, nickel-titanium alloys, tantalum-titanium alloys, nickel-aluminum alloys, inconel, gold, platinum, iridium, tungsten, tungsten alloys, cobalt alloys, etc. Among the stainless steels, preferred is SUS316L which is the best in corrosion resistance. Among the cobalt alloys, preferred are MP35N and L605 and the like. Among the tungsten alloys, preferred are W-Rh25% and W-Rh26%.

The stent body according to the present invention can be suitably formed from any of the above-mentioned materials that is selected, as required, according to the site of application of the stent and to the expanding means. For example, where the stent body is formed from a metallic material, the excellent strength of the metallic material permits the stent to be implanted securely in a lesion portion. Where the stent body is formed from a polymer material, the excellent flexibility of the polymer material permits the stent to exhibit an excellent effect in terms of the performance of arrival of the stent at a lesion portion (delivery performance).

In addition, where the stent body is produced by use of a biodegradable polymer such as polylactic acid, the stent itself disappears by being decomposed in and absorbed into the living body after functioning as a stent, specifically, after suppressing the rates of blood vessel occlusion and restenosis in an acute phase; thus, the stent body exhibits an excellent effect in terms of lowering the risk of restenosis or thrombotic complication in a late phase.

Besides, where the stent is of the self-expanding type, a restoring force for returning to the original shape is needed and, therefore, superelastic alloys (e.g., titanium-nickel alloy) and the like are preferred as the material of the stent body. Where the stent is of the balloon-expanding type, it is preferable that the possibility of shape return after expansion is low and, therefore, stainless steels and cobalt alloys are preferred as the material of the stent body.

In addition, where the stent body is formed from carbon fiber, it has both a high strength and excellent flexibility, and is excellent in safety in the living body.

The size of the stent body in the present invention may be suitably selected according to the site of application of the stent. For example, where the stent is to be used in a coronary artery of the heart, normally, the stent body before expansion preferably has an outer diameter of 1.0 to 3.0 mm and a length of 5 to 50 mm. Where the stent body is composed of a filamentous member as shown in FIG. 1, the length in the width direction of the filamentous member constituting the stent body so that the stent body has a large number of cutouts is preferably 0.01 to 0.5 mm, more preferably 0.05 to 0.2 mm.

The method for manufacturing the stent body according to the present invention is not particularly limited, and may be suitably selected from ordinarily employed manufacturing methods, according to the structure and material of the stent. For example, the method can be selected from manufacturing methods based on the utilization of an etching technique such as laser etching and chemical etching and a laser cutting technique.

In addition, the whole part or a part of the surface of the stent body in the present invention may be pre-treated to form a base coat layer, in order to enhance the adhesion between the stent body and the layer 3. However, the stent according to the present invention can have a configuration in which the stent body is coated with the layer 3 without forming any base coat layer therebetween.

Incidentally, the term "base coat layer" used herein means a layer which is formed, by a pre-treatment, so as to function as an intermediate for enhancing the adhesive force between the stent body and the above-mentioned layer containing the citric acid ester employed as a plasticizer as well as the physiologically active substance and the biodegradable polymer, before the formation of the above-mentioned layer on the stent body for the purpose of augmenting the adhesion between the above-mentioned layer and the stent body. When the layer 3 is coated to the stent body without forming such a base coat layer, it is possible to obviate the toxicity and inflammation-causing property which might arise from the material for forming the base coat layer.

Examples of the pre-treatment include a method in which a material having affinity for both the stent body and the layer 3 is coated to the surface of the stent body to form the base coat layer. As the material for forming the base coat layer, various materials can be used, of which the most preferred are silane coupling agents which have both a hydrolysable group and an organic functional group. The silanol group formed by decomposition of the hydrolysable group (e.g., alkoxy group) of the silane coupling agent can be bonded to the metallic stent body through a covalent bond, whereas the organic functional group (e.g., epoxy group, amino group, mercapto group, vinyl group and methacryloxy group) of the silane coupling agent can be bonded to the biodegradable polymer in the layer 3 through a chemical bond. Specific examples of the silane coupling agent include y-aminopropylethoxysilane and γ - glycidoxypropylmethyldimethoxysilane. Examples of the base coat material, other than the silane coupling agents, include organotitanium coupling agents, aluminium coupling agents, chromium coupling agents, organophosphoric acid coupling agents, organic vapor deposition films of polyparaxylene or the like, cyanoacrylate adhesives, and polyurethane paste resins.

The layer 3 of the stent according to the present invention includes the composition containing the physiologically active substance, the biodegradable polymer, and the citric acid ester employed as a plasticizer. It is to be noted here, however, that the layer 3 may not necessarily cover the whole surface of the filamentous member 2 constituting the stent body, and it suffices for the layer 3 to cover at least a part of the surface of the filamentous member 2 constituting the stent body. Therefore, the layer 3 may cover only the upper surface of the outside surface 21 of a sectional shape shown in FIG. 3 (the inside surface 22 is forming a short arc, whereas the outside surface 21 is forming a somewhat longer arc). In the case of the layer 3 formed in such a shape, the physiologically active substance is locally released from the stent surface into the living body tissue, so that an effective therapy can be achieved.

The physiologically active substance in the present invention is not particularly limited, and can be selected arbitrarily, insofar as the substance will restrain the stenosis or occlusion of a systema vasorum which might occur when the stent according to the present invention is implanted in a lesion portion. For example, the physiologically active substance may be at least one substance selected from the group including carcinostatic agents, immunosuppressants, antibiotics, antirheumatics, antithrombotic agents, HMG-CoA reductase inhibitors, ACE inhibitors, calcium antagonists, antihyperlipidemic agents, integrin inhibitors, antiallergic agents, antioxidants, GPIIbIIIa antagonists, retinoids, flavonoids, carotenoids, lipid improving drugs, DNA synthesis inhibitors, tyrosine kinase inhibitors, antiplatelet agents, anti-inflammatory agents, bio-derived materials, interferons, and NO production promoting substances, which are preferable because they make it possible to control the behavior of the cells of the tissue of a lesion portion and to treat the lesion portion.

Preferable examples of the carcinostatic agents include vincristine, vinblastine, vindesine, irinotecan, pirarubicin, paclitaxel, docetaxel, and methotrexate.

Preferred examples of the immunosuppressants include sirolimus, sirolimus derivative such as everolimus, pimecrolimus, ABT-578, AP23573, CCI-779 and the like, tacrolimus, azathioprine, ciclosporin, cyclophosphamide, mycophenolate mofetil, gusperimus, and mizoribine, among which more preferred are sirolimus, sirolimus derivative such as everolimus, pimecrolimus, ABT-578, AP23573, CCI-799 and the like, and tacrolimus.

Preferable examples of the antibiotics include mitomycin, adriamycin, doxorubicin, actinomycin, daunorubicin, idarubicin, pirarubicin, aclarubicin, epirubicin, peplomycin, and zinostatin stimalamer.

Preferred examples of the antirheumatics include methotrexate, sodium thiomalate, penicillamine, and lobenzarit.

Preferable examples of the antithrombotic agents include heparin, aspirin, antithrombin preparation, ticlopidine, and hirudin.

Preferred examples of the HMG-CoA reductase inhibitors include cerivastatin, cerivastatin sodium, atorvastatin, rosuvastatin, pitavastatin, fluvastatin, fluvastatin sodium, simvastatin, lovastatin, and pravastatin.

Preferable examples of the ACE inhibitors include quinapril, perindopril erbumine, trandolapril, cilazapril, temocapril, delapril, enalapril maleate, lisinopril, and captopril.

Preferred examples of the calcium antagonists include hifedipine, nilvadipine, diltiazem, benidipine, and nisoldipine.

Preferable examples of the antihyperlipidemic agents include probucol.

Preferred examples of the integrin inhibitors include AJM300.

Preferable examples of the antiallergic agents include tranilast.

Preferred examples of the antioxidants include α-tocopherol.

Preferable examples of the GPIIbIIIa antagonists include abciximab.

Preferred examples of the retinoids include all-trans-retinoic acid.

Preferable examples of the flavonoids include epigallocatechin, anthocyanine, and proanthocyanidin.

Preferred examples of the carotenoids include β-carotene, and lycopene.

Preferable examples of the lipid improving drugs include eicosapentaenoic acid.

Preferred examples of the DNA synthesis inhibitors include 5-FU.

Preferable examples of the tyrosine kinase inhibitors include genistein, tyrphostin, erbstatin, and staurosporine.

Preferred examples of the antiplatelet agents include ticlopidine, cilostazol, and clopidogrel.

Preferable examples of the antiinflammation agents include steroid such as dexamethasone, prednisolone and the like.

Preferable examples of the bio-derived materials include EGF (epidermal growth factor), VEGF (vascular endothelial growth factor), HGF (hepatocyte growth factor), PDGF (platelet derived growth factor), and BFGF (basic fibrolast growth factor).

Preferred examples of the interferons include interferon- γ1a.

Preferable examples of the NO production promoting substances include L-arginine.

The layer 3 may contain only one kind of physiologically active substance or two or more kinds of physiologically active substances, of the above-mentioned physiologically active substances. Where the layer 3 contains two or more kinds of physiologically active substances, the combination may be made by appropriately selecting from among the above-mentioned physiologically active substances, as required. The physiologically active substances according to the present invention are preferably sirolimus, sirolimus derivative such as everolimus, pimecrolimus, ABT-578, AP23573, CCI-779 and the like, and tacrolimus, more preferably sirolimus or a sirolimus derivative.

The biodegradable polymer in the present invention is not particularly limited, insofar as it is a polymer which can be gradually biodegraded when the stent of the present invention is implanted in a lesion portion and it is a polymer which does not adversely effect the living body of a human being or an animal. Preferably, the biodegradable polymer is at least one polymer selected from the group including aliphatic polyesters, polyesters, polyacid anhydrides, polyorthoesters, polycarbonates, polyphosphazenes, polyphosphoric acid esters, polyvinyl alcohol, polypeptides, polysaccharides, proteins, and cellulose, a copolymer in which monomers constituting the polymers are copolymerized as desired, or a mixture of the polymer(s) and/or the copolymer(s). Incidentally, the term "mixture" herein means a wide concept inclusive of complex such as polymer alloys. Among these biodegradable polymers, aliphatic polyesters are preferred because they are low in reactivity for reaction with living body tissues and their decomposition in the living body can be controlled.

The aliphatic polyesters are not particularly limited. Preferably, the aliphatic polyester is at least one polymer selected from the group including polylactic acid, polyglycolic acid, polyhydroxylactic acid, polyhydroxyvaleric acid, polyhydroxypentanoic acid, polyhydroxyhexanoic acid, polyhydroxyheptanoic acid, polycaprolactone, polytrimethylene carbonate, polydioxanone, polymalic acid, polyethylene adipate, polyethylene succinate, polybutylene adipate, and polybutylene succinate, a copolymer in which monomers constituting the polymers are copolymerized as desired, or a mixture of the polymer(s) and/or the copolymer(s). Among these aliphatic polyesters, polylactic acid and polyglycolic acid are further preferable because they are high in bio-compatibility and their decomposition in the living body can be controlled easily.

The polylactic acid means polylactic acid or a copolymer of lactic acid with a hydroxycarboxylic acid. Preferable examples of the hydroxycarboxylic acid include glycolic acid, caprolactone, trimethylene carbonate, and dioxanone. These lactic acid resins can each be obtained by selecting compounds of desired structures from among L-lactic acid, D-lactic acid and hydroxycarboxylic acids as raw materials, and subjecting the raw materials to dehydration polymerization. Preferably, the lactic acid resin can be obtained by selecting compounds of desired structures from among lactides, which are cyclic dimers of lactic acid, glycolides, which are cyclic dimers of glycolic acid, and caprolactone, etc., and subjecting the selected compounds to ring opening polymerization. The lactides include L-lactide, which is a cyclic dimer of L-lactic acid, D-lactide, which is a cyclic dimer of D-lactic acid, meso-lactide, which is obtained by cyclic dimerization of D-lactic acid with L-lactic acid, and DL-lactide, which is a racemic compound of D-lactide with L-lactide. In the present invention, any of these lactides can be used.

Incidentally, where the above-mentioned aliphatic polyester is selected as the biodegradable polymer in the present invention, it is preferably polylactic acid (PLA), polyglycolic acid (PGA), lactic acid-glycolic acid copolymer (PLGA), or a copolymer of them.

An embodiment of production of the stent according to the present invention (the embodiment will hereinafter be referred to as "the embodiment of the present invention") will be described below.

In the embodiment of the present invention, the layer 3 including the composition containing the physiologically active substance, the biodegradable polymer and the citric acid ester is formed on the surface of the stent body. It is to be noted here that the layer 3 may not necessarily be so formed as to cover the whole surface of the stent body and may be so formed as to cover at least a part of the surface of the stent body. Therefore, a configuration may be adopted in which only the outside surface of the stent body having a hollow cylindrical form is coated with the layer 3. The method for forming the layer 3 on the surface of the stent body is not particularly limited, insofar as it is possible to form the layer 3 as above-mentioned and to ensure that, when the stent is implanted in a lumen in a living body, the physiologically active substance loaded in the layer 3 can be released sustainedly. Specifically, a method may be adopted in which the physiologically active substance, the biodegradable polymer and the citric acid ester in the above-mentioned ratio are dissolved in a solvent such as acetone, ethanol, chloroform and tetrahydrofuran so as to attain a solution concentration (the total concentration of the physiologically active substance, the biodegradable polymer and the citric acid ester in the solvent) of 0.001 to 20 mass%, preferably 0.01 to 15 mass%, the resulting solution is spread to the whole surface or a part of the surface of the filamentous member 2 constituting a stent body by use of a spray, or a dispenser, an ink jet, a spray or the like capable of ejecting a tiny amount of the solution, and then the solvent is evaporated off. Or, alternatively, a method may be adopted in which the stent body is immersed in the above-mentioned solution, and then the solvent is evaporated off.

The means of expanding the stent 1 according to the present invention produced by such a method is not particularly limited, and may be the same as in ordinary stents. For example, the stent may be of the self-expanding type, namely, the type such that when a force for holding the stent in a minutely folded state is removed, the stent will expand in the radial direction by its own restoring force. It is to be noted here, however, that the stent 1 according to the present invention is preferably of the balloon-expanding type, namely, the type such that the stent is expanded in the radial direction under an external force exerted thereon by dilating a balloon set inside the stent body.

Now, one embodiment of the method of producing the stent according to the present invention will be described below, but the scope of the invention is not to be limited to this mode.

### <Method of Producing Stent>

### "Method in which Biodegradable Polymer is used as Material of Stent Body"

A physiologically active substance in the present invention is dissolved in a solvent such as acetone and THF, and the resulting solution is mixed with a biodegradable polymer and a citric acid ester in predetermined concentrations, to prepare a composition. In mixing the physiologically active substance, the biodegradable polymer and the citric acid ester in the present invention (the physiologically active substance, the biodegradable polymer and the citric acid ester in the present invention will be referred to also as the components in the present invention), a kneading apparatus or the like may be used. The apparatus for kneading the physiologically active substance, the biodegradable polymer and the citric acid ester in the present invention is not particularly limited, insofar as the above-mentioned each component can be subjected to shear kneading. Examples of the kneading apparatus include extruders, Bumbury's mixer, rollers, and kneaders.

Examples of the extruders include screw extruders such as single-axial screw extruder and biaxial screw extruder, elastic extruder, hydrodynamic extruder, ram type extruder, roller type extruder, gear type extruder, etc. Among these extruders, preferred is the screw extruder, particularly, the biaxial screw extruder, and more preferred is a biaxial screw extruder provided with at least one vent (deaeration port) which is excellent in deaeration efficiency. The order in which the components are kneaded is not particularly limited. The temperature at the time of kneading is not lower than the glass transition point, and lower than the melting temperature, of the physiologically active substance, the biodegradable polymer or the citric acid ester in the present invention. Where the kneading temperature is within such a range, the components in the present invention are softened, whereby the load on the kneading apparatus can be reduced, which naturally is preferable. Incidentally, the melting temperature herein means the end point temperature of an absorption peak of heat of fusion of crystal which appears at the time of temperature rise measurement by use of a differential scanning calorimeter (DSC). Besides, the glass transition point herein means a transition temperature determined from a thermograph obtained upon a measurement conducted by use of a differential scanning calorimeter according to JIS-K7121.

Incidentally, the solvent is not limited to the above-mentioned, and any solvent that can dissolve the physiologically active substance in the present invention can be employed in the present invention.

The physiologically active substance in the present invention is dissolved in the solvent such as acetone and THF, the resulting solution is mixed with the biodegradable polymer and the citric acid ester in predetermined concentrations to prepare a composition, and the composition is formed into a pipe having a predetermined material thickness and a predetermined outer shape by use of an injection molding machine, an extrusion molding machine, a press forming machine, a vacuum forming machine, a blow molding machine or the like. Then, an aperture pattern is adhered to the surface of the pipe, and the pipe portions other than the aperture pattern are melted by an etching technique such as laser etching and chemical etching to form apertures in the pipe wall. Or, alternatively, the pipe may be cut according to a pattern by a laser cutting technique based on pattern data stored in a computer, whereby apertures can be formed in the pipe wall.

Other stent than the above-mentioned, for example, a coil-formed stent can be produced as follows. The physiologically active substance in the present invention is dissolved in the solvent such as acetone and THF, the resulting solution is mixed with the biodegradable polymer and the citric acid ester in predetermined concentrations to prepare a composition, and the composition is formed into a wire having a predetermined diametral size and a predetermined outer shape by use of an extrusion molding machine. Then, the wire is bent into a pattern such as a wavy pattern, and the patterned wire is wound helically around a mandrel, then the mandrel is pulled out, and the thus shaped wire is cut to a predetermined length.

### "Method in which Metallic Material is used as Material of Stent Body"

First, a composition containing the physiologically active substance, the biodegradable polymer and the citric acid ester in the present invention is dissolved in a solvent such as acetone, and the resulting solution is spread or misted to predetermined positions of the surface of the stent body including a filamentous member by use of a conventional method using a spray, a dispenser or the like, or by immersing the stent body in the solution. Thereafter, the solvent is evaporated off.

Or, alternatively, the physiologically active substance in the present invention is dissolved in a solvent such as acetone, the resulting solution is spread to at least a part of the stent body made of material such as stainless steel by a conventional method using a spray, a dispenser or the like, and then the solvent is evaporated off. Next, the biodegradable polymer and the citric acid ester in the present invention are dissolved in acetone or the like in the same manner as above, the resulting solution is spread onto the layer of the physiologically active substance by the same method as above, and then the solvent is evaporated off, to obtain a stent having a structure in which a layer of a mixture of the biodegradable polymer and the citric acid ester is formed on the layer of the physiologically active substance.

Here, before the spreading of the physiologically active substance in the present invention, the biodegradable polymer in the present invention and the mixture thereof to the stent body, an operation for forming minute concavity and convexity in the surface of the stent body in the present invention is preferably carried out through etching, for example, by subjecting the surface to a chemical treatment, a plasma or the like. This enhances the adhesion between the stent body in the present invention and the physiologically active substance in the present invention, the biodegradable composition in the present invention, and the mixture thereof. For the same reason as above, an adhesive or the like may be applied to the surface of the stent body in the present invention; alternatively, the stent body in the present invention, the physiologically active substance in the invention, the biodegradable polymer in the invention, and the mixture thereof may be thermal adhered to each other. By such a method, the stent according to the present invention can be produced.

Incidentally, as the stent body, any of known stent bodies can be used suitably. For example, the stent bodies produced according to the structures and methods as disclosed in US-B-6183508, WO 94/12136, US-A-5716396, WO 96/03092, US-A-5716396, WO 95/03010, JP-B-1991-68939, etc. may be used. As for the structure of the stent body, more specifically, there can be used a stent body produced by a method in which an elastic filamentous member is bent into a coil-like form and a plurality of the coil-like members are connected to form a hollow cylindrical body having cutouts including clearances between the elastic filamentous members; a stent body produced by a method in which an elastic filamentous member is bent into the shape of a snake-like flat ribbon, the ribbon is wound helically around a mandrel to obtain a hollow cylindrical body having cutouts including clearances between the elastic filamentous members; a stent body having a mesh-formed structure in which the shape of cutouts is a meander pattern shape; a stent body produced by a method in which a plate-formed member is bent into a coil-like form to obtain a hollow cylindrical body having cutouts including clearances between the adjacent coil portions; a stent body produced by a method in which elastic plate-formed member is bent like a whirl to form a hollow cylindrical body not having any cutout in the side surface thereof.

### Examples

Now, the present invention-will be described more in detain below, based on non-limitative examples.

(Reference Example 1) (This example is made to be a reference example, as a result of investigations and based on the remark pointing out that it is undesirable to broaden the scope of claim 1 or to broaden the description of the specification to thereby substantially broaden claim 1.)
First, in order to confirm the effect of the plasticizer, a cast film was prepared, and it was served to a tensile test.

First, 250 mg of polylactic acid (PLA) (Resomer R203, produced by Boehringer-Ingelheim) or a lactic acid-glycolic acid copolymer (PLGA) (Resomer RG504, produced by Boehringer-Ingelheim) as a biodegradable polymer and 50 mg (20 parts by mass based on 100 parts by mass of polylactic acid) of triethyl citrate (produced by Wako Pure Chemical Industries, Ltd.) or acetyltributyl citrate (produced by Wako Pure Chemical Industries, Ltd.) or butyryltrihexyl citrate (produced by Wako Pure Chemical Industries, Ltd.) as a plasticizer, were dissolved in 5 mL of acetone, the resulting solution was poured into a Teflon-made Petri dish 50 mm in diameter, and the solution was dried at room temperature for two days and in vacuum for one day, to prepare a cast film. As a comparative example, a cast film without addition of any plasticizer was also prepared in the same manner as above. The thickness of the films was 150 to 180 µm.

Each of the cast films was blanked into a dumb-bell shape of No. 2 small specimen (a size of 1/5 times that of No. 2 specimen) described in JIS K7113, and the specimen was served to a tensile test according to JIS K7113 (1995) by use of a high-precision universal testing machine (Autograph AGS-1KING, produced by Shimadzu Corporation). The results are shown in Table 1 below.

**[Table 1]**

| Plasticizer | Breaking elongation in tension (%) | Modulus of elasticity in tension (MPa) |
|---|---|---|
| PLA/Triethyl citrate | 428 | 18 |
| PLA/Acetyltributyl citrate | 684 | 20 |
| PLA/Butyryltrihexyl citrate | 617 | 16 |
| PLA alone | 20 | 2080 |
| PLGA/Triethyl citrate | 389 | 16 |
| PLGA/Acetyltributyl citrate | 659 | 18 |
| PLGA/Butyryltrihexyl citrate | 536 | 17 |
| PLGA alone | 10 | 1720 |

It is seen from the results given in Table 1 above that the films prepared by adding triethyl citrate, acetyltributyl citrate or butyryltrihexyl citrate were remarkably enhanced in elongation (breaking elongation in tension) and flexibility (modulus of elasticity in tension), as compared with the film prepared without addition of citric acid ester (Reference Example). The results imply that, where the stent is coated with a coating layer formed by addition of citric acid ester, there is low possibility of breakage of the coating layer at the time of expanding the stent.

### <Example 1>

First, 50 mg of sirolimus (Rapamycin, produced by SIGMA) which is an immunosuppressant as a physiologically active substance, 50 mg of polylactic acid (PLA) (Resomer R203, produced by Boehringer-Ingelheim) as a biodegradable polymer, and 10 mg (20 parts by mass based on 100 parts by mass of polylactic acid) of acetyltributyl citrate (ATBC) (produced by Wako Pure Chemical Industries, Ltd.) were dissolved in 1 mL of acetone, to prepare a coating solution.

By use of a micro-dispenser, the coating solution was spread to the outside surface of a filamentous member 2 (width: 0.1 mm) constituting a stent body (material: SUS316L) having a hollow cylindrical shape with an outer diameter of 2.0 mm, a thickness of 100 µm and a length of 30 mm and provided with roughly rhombic cutouts as shown in FIG. 1. Thereafter, acetone was completely evaporated off by vacuum drying, to form a coating layer having a mean thickness of 10 µm.

The stent was mounted on a balloon of a delivery catheter, the balloon was dilated so as to expand the stent up to 3.0 mm in an outer diameter, and the outside surface of the stent was observed by use of a CCD camera. As a result, there was observed no damaged portion having crack in the coating layer or exfoliation of the coating layer from the stent body (FIG. 4).

### <Example 2>

A coating solution was prepared by dissolving 50 mg of a lactic acid-glycolic acid copolymer (PLGA) (Resomer RG504, produced by Boehringer-Ingelheim) as a biodegradable polymer, 50 mg of sirolimus which is an immunosuppressant as a physiologically active substance, and 10 mg (20 parts by mass based on 100 parts by mass of polylactic acid) of acetyltributyl citrate (ATBC) as a plasticizer in 1 mL of acetone. The coating solution was spread to a stent body by the same manner as that described in Example 1 above, and the stent thus obtained was tested.

The stent was mounted on a balloon of a delivery catheter, the balloon was dilated so as to expand the stent up to 3.0 mm in an outer diameter, and then the outside surface of the stent was observed by use of CCD camera. As a result, there was observed no damaged portion having crack in the coating layer or exfoliation of the coating layer from the stent body (FIG. 5).

### <Example 3>

A coating solution was prepared by dissolving 50 mg of polylactic acid (PLA) (Resomer R203, produced by Boehringer-Ingelheim) as a biodegradable polymer, 50 mg of sirolimus which is an immunosuppressant as a physiologically active substance, and 10 mg (20 parts by mass based on 100 parts by mass of polylactic acid) of butyryltrihexyl citrate (BTHC) (produced by Wako Pure Chemical Industries, Ltd.) as a plasticizer in 1 mL of acetone. The coating solution was spread to a stent body by the same method as that described in Example 1 above, and the stent thus obtained was tested.

The stent was mounted on a balloon of a delivery catheter, the balloon was dilated so as to expand the stent up to 3.0 mm in an outer diameter, and the outside surface of the stent was observed by use of a CCD camera. As a result, there was observed no damaged portion having crack in the coating layer or exfoliation of the coating layer from the stent body (FIG. 6).

### <Example 4>

A coating solution was prepared by dissolving 50 mg of a lactic acid-glycolic acid copolymer (PLGA) (Resomer RG504, produced by Boehringer-Ingelheim) as a biodegradable polymer, 50 mg of sirolimus which is an immunosuppressant as a physiologically active substance, and 10 mg (20 parts by mass based on 100 parts by mass of polylactic acid) of butyryltrihexyl citrate (BTHC) (produced by Wako Pure Chemical Industries, Ltd.) as a plasticizer in 1 mL of acetone. The coating solution was spread to a stent body by the same method as that described in Example 1 above, and the stent thus obtained was tested.

The stent was mounted on a balloon of a delivery catheter, the balloon was dilated so as to expand the stent up to 3.0 mm in an outer diameter, and the outside surface of the stent was observed by use of a CCD camera. As a result, there was observed no damaged portion having crack in the coating layer or exfoliation of the coating layer from the stent body (FIG. 7).

### comparative Example 1>

A stent was produced by use of polylactic acid (PLA) in the same manner as in Examples 1 and 3, except that no plasticizer was added. The stent was mounted on a balloon of a delivery catheter, the balloon was dilated so as to expand the stent up to 3.0 mm in an outer diameter, and the outside surface of the stent was observed by use of a CCD camera. As a result, there were observed many damaged portions having crack in the coating layer, exfoliation of the coating layer from the stent body, which was probably brought about at the time of dilating the balloon (FIG. 8).

### <Comparative Example 2>

A stent was produced by use of a lactic acid-glycolic acid copolymer (PLGA) in the same manner as in Examples 2 and 4, except that no plasticizer was added.

The stent was mounted on a balloon of a delivery catheter, the balloon was dilated so as to expand the stent up to 3.0 mm in an outer diameter, and the outside surface of the stent was observed by use of a CCD camera. As a result, there were observed many damaged portions having crack in the coating layer, exfoliation of the coating layer from the stent body, which was probably brought about at the time of dilating the balloon (FIG. 9).

### <Example 5>

A test was conducted in which each of the stents produced in Example 1 (PLA/ATBC) and Example 2 (PLGA/ATBC) was implanted into a swine coronary artery. The stent mounted on a balloon of a delivery catheter was inserted through the right carotid artery of swine to deliver the stent to an about 3 mm diameter portion of the swine coronary artery, and the balloon was dilated by applying a 10 atm water pressure to the balloon so that the ratio of the dilated balloon diameter to the blood vessel diameter observed by contrast study became about 1.1, thereby implanting the stent in the swine coronary artery. As a result, the values of stenosis rate measured one month after the implantation were 20% and 19%, respectively.

Upon autopsy and pathologic evaluation conducted one month after the implantation, no inflammation reaction was observed at the portion where the stent had been implanted, and it was observed that hypertrophy due to multiplication of smooth muscle cells had been suppressed (FIG. 10: PLA/ATBC, FIG. 11: PLGA/ATBC).

### <Comparative Example 3>

A test of implanting a stainless steel-made stent (material: SUS316L, outer diameter: 1.8 mm, length: 15 mm, thickness: 80 µm) into a swine coronary artery was conducted in the same manner as in Example 5 above, for the purpose of comparison of the stent according to the present invention with a stent not provided with any coating layer in therapeutic effect.

As a result, the stenosis rate measured one month after the implantation was 30%.

Upon autopsy and pathologic evaluation conducted one month after the implantation, no inflammation reaction was observed at the portion where the stent had been implanted, but it was observed that hypertrophy due to multiplication of smooth muscle cells had occurred (FIG. 12).

Furthermore, the present application is based on Japanese Patent Application No. 2006-014365 filed January 23, 2006, and the subject matter disclosed in the patent application is referred to and entirely incorporated herein.

## Claims

1. A stent to be implanted in a lumen in a living body, wherein a layer comprised of a composition containing a physiologically active substance, a biodegradable polymer and a citric acid ester employed as a plasticizer is formed at least at a part of the surface of a stent body.

2. The stent according to claim 1, wherein no base coat layer is present between said stent body and said layer which is comprised of said composition and formed at least at a part of the surface of said stent body.

3. The stent according to claim 1 or 2, wherein said citric acid ester employed as said plasticizer is at least one compound selected from the group including triethyl citrate, tributyl citrate, acetyltriethyl citrate, acetyltributyl citrate, trihexyl citrate, acetyltrihexyl citrate, butyryltrihexyl citrate, and analogs thereof.

4. The stent according to any one of claims 1 to 3, wherein said citric acid ester employed as said plasticizer is contained in an amount in the range of 10 to 40 parts by mass based on 100 parts by mass of said biodegradable polymer.

5. The stent according to any one of claims 1 to 4, wherein said biodegradable polymer is at least one polymer selected from the group including aliphatic polyesters, polyesters, polyacid anhydrides, polyorthoesters, polycarbonates, polyphosphazenes, polyphosphoric acid esters, polyvinyl alcohol, polypeptides, polysaccharides, proteins, and cellulose, a copolymer in which monomers constituting said polymers are copolymerized as desired, or a mixture of said polymer(s) and/or said copolymer(s).

6. The stent according to any one of claims to 5, wherein said biodegradable polymer is an aliphatic polyester.

7. The stent according to claim 5 or 6, wherein said aliphatic polyester is at least one selected from the group including polylactic acid (PLA), polyglycolic acid (PGA), and lactic acid-glycolic acid copolymer (PLGA).

8. The stent according to any one of claims 1 to 7, wherein said physiologically active substance is at least one compound selected from the group including carcinostatic agents, immunosuppressants, antibiotics, antirheumatics, antithrombotic agents, HMG-CoA reductase inhibitors, ACE inhibitors, calcium antagonists, antihyperlipidemic agents, integrin inhibitors, antiallergic agents, antioxidants, GPIIbIIIa antagonists, retinoids, flavonoids, carotenoids, lipid improving drugs, DNA synthesis inhibitors, tyrosine kinase inhibitors, antiplatelet agents, anti-inflammatory agents, bio-derived materials, interferons, and NO production promoting substances.

9. The stent according to any one of claims 1 to 8, wherein said physiologically active substance is sirolimus or a sirolimus derivative.

10. The stent according to any one of claims 1 to 9, wherein said stent body is made of a metal or a polymer.
